(19) Europäisches Patentamt
European Patent Office
Office européen des brevets

(11) Publication number: **0 074 693**
**B1**

(12) **EUROPEAN PATENT SPECIFICATION**

(45) Date of publication of patent specification: **08.01.86**

(21) Application number: **82201122.7**

(22) Date of filing: **08.09.82**

(51) Int. Cl.⁴: **C 07 C 49/427,**
C 07 C 49/623, A 61 K 7/46,
C 11 B 9/00 // C07C45/68,
C07C45/62, C07C45/72

(54) **Perfume compositions and perfumed articles containing spiro-undecanones and -undecenones as perfume base.**

(30) Priority: **16.09.81 NL 8104271**

(43) Date of publication of application:
**23.03.83 Bulletin 83/12**

(45) Publication of the grant of the patent:
**08.01.86 Bulletin 86/02**

(84) Designated Contracting States:
**CH DE FR GB LI NL**

(56) References cited:
**US-A-4 052 457**

**JOURNAL OF MEDICINAL CHEMISTRY, vol. 8, November 1965, pp. 825-9; L.M.RICE et al.: "Spiranes. X. Aminospiranes"**

(73) Proprietor: **NAARDEN INTERNATIONAL N.V.**
**P.O.Box 2 Huizerstraatweg 28**
**NL-1400 CA Naarden-Bussum (NL)**

(72) Inventor: **van der Weerdt, Antonius J.A.**
**Hinde 11**
**NL-1273 GN Huizen (NL)**
Inventor: **Apeldoorn, Willem**
**de Dam 36**
**NL-1261 KT Blaricum (NL)**

(74) Representative: **van der Beek, George Frans et al**
**Nederlandsch Octrooibureau Johan de Wittlaan 15 P.O. Box 29720**
**NL-2502 LS 's-Gravenhage (NL)**

**EP 0 074 693 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to perfume compositions containing spiro-undecanone derivatives and spiro-undecenone derivatives as perfume base, to articles perfumed with these compounds respectively with perfume compositions containing these compounds and to new spiro-undecanones.

There is a continuing interest in the preparation and application of synthetic fragrance materials because contrary to materials of natural origin synthetic materials can always be prepared in the quantity desired and with uniform quality. In particular there is a large demand for synthetic fragrance materials having a natural odour character moreover, for certain applications like perfuming of modern detergents, a high chemical stability is required.

It has been found that derivatives of spiro[5.5]undecane-3-one and [5.5]-undecene-3-one of the formula

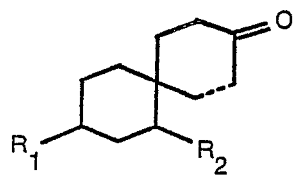

(1)

in which $R_1$ and $R_2$ represent a hydrogen atom or a methyl group and the dotted line is a carbon-carbon single or double bond, provided that when this dotted line is a double bond $R_1$ and $R_2$ are both hydrogen atoms are valuable fragrance materials having somewhat fruity and spicy odours which also strongly resemble orris.

Only little is known about the olfactory properties of spiro ketones. However, in US patent specification No. 3,923,837 it is mentioned that several 6.10-dialkyl-spiro[4.5]-decane-2-ones and [4.5]decane-8-ones and corresponding dec-6-enones are useful as fragrance and flavour materials and are characterized by woody, earthy and balsamic odour notes remembering of ambergris, ylang, sandalwood and patchouli. Further, US patent specification No. 4,203,925 discloses a number of alkyl substituted spiro[4,5]decane-8-ones, -decene-8-ones and -decadiene-8-ones, which are related to ß-vetivone (2-isopropylidene-6,10-dimethyl-spiro[4,5]dec-6-ene-8-one), a component of vetiver oil. These compounds also have woody and earthy odours. So from a chemical point of view as well as with respect to the olfactory properties the compounds disclosed in the above mentioned US patent specifications are very different from the compounds according to the present invention.

It is emphasized that the use of derivatives of spiro-undecane and -undecene and in particular of spiro [5,5] undecane and -undecene is completely unknown in the perfume industry. On the other hand the undermentioned compounds having the formulas 1a, 1b and 1c are known per se and have been described in the cited literature. So no exclusive rights are claimed for these compounds as such. Further no olfactory properties of these known compounds are known.

1a) Spiro[5.5]undecane-3-one

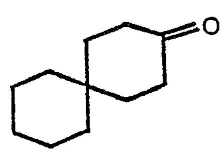

(1a)

H.A.P. de Jongh and H. Wijnberg, Tetrahedron *20*, 2553—73 (1964);
British patent specifications 1,343,915 and 1,343,916.

1b) Spiro[5,5]undec-1-ene-3-one.

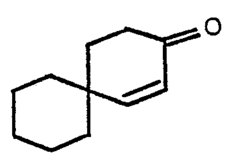

(1b)

H. Cristol, F. Plenat and J. Salancon, Bull. Soc. Chim. France *1970*, 4468—71;
V. V. Kane, Synth. Commun. *6*, 237—42 (1976);
S. F. Martin, J. Org. Chem. *41*, 3337—8 (1976).

1c) 9-Methyl-spiro[5.5]undecane-3-one.

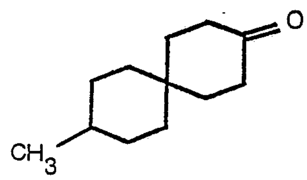

(1c)

L. M. Rice, E. C. Dobbs and C. H. Grogan, J. Med. Chem. *8*, 825—9 (1965) vide Chem. Abstract *63* 16227e.

The compounds 7-methyl-spiro[5.5]undecane-3-one having the formula

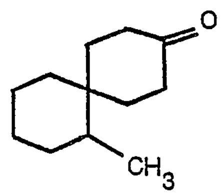

(1d)

and 7.9-dimethyl-spiro[5.5]undecane-3-one having the formula

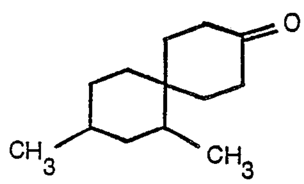

(1e)

are new.

The compounds according to the invention can be prepared according to the methods described in the literature, especially in the references mentioned for the compound having formula (1b). The best-known method is illustrated in the following reaction scheme:

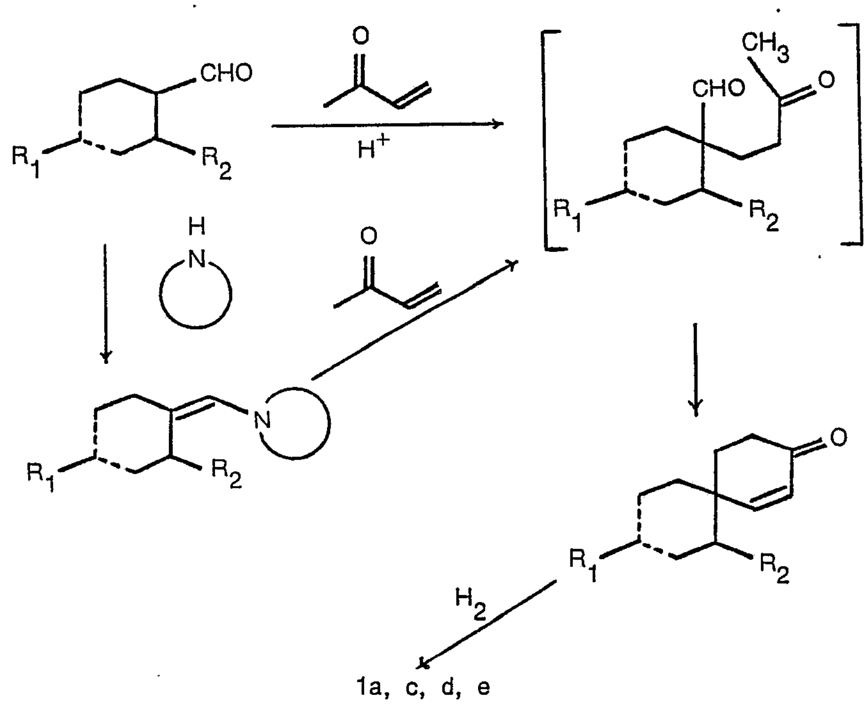


in which the dotted lines represent single bonds or at most one double bond. According to this method a suitable cyclohexene- or cyclohexane carbaldehyde, optionally as an enamine, is reacted with methyl phenyl ketone followed by cyclisation of the keto-aldehyde in an intramolecular aldol condensation. This last step often proceeds spontaneously. When the route via the enamine is followed, every suitable secondary amine can be used. Amines which are often used for such reactions are for instance pyrrolidine

and morpholine. The double bonds can be removed from the reaction product acording to known methods such as catalytic hydrogenation.

The spiroketones according to the invention are powerful fragrance materials having a very natural fruity, spicy odour reminiscent of orris. Especially for the compounds of formulae 1b, 1d and 1e the orris character is very pronounced whereas the compound of formula 1a has a somewhat stronger fruity-spicy character. The spiroketones according to the invention can be used successfully in perfume compositions or as such as odour imparting agents. Because of their chemical stability the compounds according to the invention are very suitable for perfuming of aggressive media like modern detergents.

The phrase "perfume composition" is used to mean a mixture of fragrance materials and optionally auxiliary substances that may be dissolved in an appropriate solvent or mixed with a powdery substrate and used to impart a desired odour to the skin and/or to various products. Examples of said products are: soaps, detergents, dish washing and cleaning agents, air fresheners and room sprays, pommanders, candles, cosmetics such as creams, ointments, colognes, pre- and after shaving lotions, talcum powders, hair care agents, body deodorants and antiperspirants.

Fragrance materials and mixtures thereof which, in combination with the compounds according to the invention, can be used for the preparation of perfume compositions include natural products such as essential oils, absolutes, resinoids, resins, concretes etc., synthetic fragrance materials, such as hydrocarbons, alcohols, aldehydes, ketones, ethers, acids, esters, acetals, ketals, nitrils etc., both saturated and unsaturated compounds, aliphatic, carbocyclic and heterocyclic compounds.

Fragrance materials which may be used in combination with the compounds according to the invention include geraniol, geranyl acetate, linalool, linalyl acetate, tetrahydrolinalool, citronellol, citronellyl acetate, myrcenol, myrcenyl acetate, dihydro myrcenol, dihydro myrcenyl acetate, tetrahydro myrcenol,terpineol, terpinyl acetate, nopol, nopyl acetate, ß-phenyl ethanol, ß-phenylethyl acetate, benzyl alcohol, benzyl acetate, benzyl salicylate, benzyl benzoate, amyl salicylate, styrallyl acetate, dimethylbenzyl carbinol, trichloromethyl-phenyl-carbinyl acetate, p-tert.butyl-cyclohexyl acetate, isononyl acetate, vetiveryl acetate, vetiverol, α-hexyl-cinnamaldehyde, 2-methyl-3-(p-tert.butylphenyl)-propanol, 2-methyl-3-(p-isopropylphenyl)-propanol, 3-(p-tert.butylphenyl)-propanol, tricyclodecenyl acetate, tricyclodecenyl propionate, 4-(4-hydroxy-4-methylpentyl)-3-cyclohexene carbaldehyde, 4-(4-methyl-3-pentenyl)-3-cyclohexene carbaldehyde, 4-acetoxy-3-pentyl-tetrahydropyran, 3-carboxymethyl-2-pentylcyclopentane, 2-n-heptyl-cyclopentanone, 3-methyl-2-pentyl-2-cyclopentanone, n-decanal, n-dodecanal, 9-decenol-1, phenoxyethyl isobutyrate, phenylacetaldehyde dimethylacetal, phenylacetaldehyde diethylacetal, geranyl nitrile, citronellyl nitrile cedryl acetate, 3-isocamphyl-cyclohexanol, cedrylmethyl ether, isolongifolanone aubepine nitrile, aubepine, heliotropine, coumarin, eugenol, vanillin, diphenyl oxide, hydroxycitronellal, ionones, methylionones, isomethylionones, irones, cis-3-hexenol and esters thereof, indan musks, tetralin musks, isochroman musks macrocyclic ketones, macrolactone musks, ethylene brassylate, aromatic nitromusks.

Auxiliary agents and solvents that may be incorporated into perfume compositions according to the invention are e.g. ethanol, isopropanol, diethyleneglycol monoethylether, diethyl phthalate etc.

The amount of the compounds of the present invention that can be used in a perfume composition or in a perfumed product can vary within broad limits and depends, for example, on the product wherein the perfume is used, the nature and the amount of the other components of the perfume compositions and the odour effect desired. Therefore, it is only possible to give rough limits. However, these limits will give a person skilled in the art sufficient information concerning the odour strength and possibilities for the use of the compounds according to the invention. In most cases a quantity of only 0.01% in a perfume composition is sufficient to obtain a clearly observable odour effect.

In some cases, however, concentrations of 30% or more may be used in the compositions to impart specific odour effects.

In products perfumed with the aid of perfume compositions according to the invention the concentration is proportionally lower depending on the quantity of the composition used in the product.

The following examples only illustrate the preparation and the use of the compounds according to the invention and do not restrict the invention thereto.

### Example I

Preparation of 7-methyl-spiro[5.5]undecane-3-one.

A solution of 186 g (1.5 mole) 6-methyl-3-cyclohexene carbaldehyde, 148 g (1.7 mole) morpholine and 500 ml toluene was refluxed under simultaneous azeotropic removal of the formed water. When all the water was distilled off the toluene was removed by evaporation. The residue was brought in a nitrogen atmosphere and then 400 ml ethanol and 119 g (1.7 mole) methylvinyl ketone were added. This mixture was refluxed for 20 hours in a nitrogen atmosphere. After the mixture was cooled down, 350 ml of a buffer solution consisting of 2 parts by weight of water, 2 parts by weight of acetic acid and 1 part by weight of sodium acetate. This mixture was refluxed for 2 hours. Then the ethanol was removed by evaporation and the residue was poured out in brine. The mixture was extracted 3 times with ether. The united ether layers were washed alkaline with a 10% soda solution and subsequently washed once with water and dried over $MgSO_4$. Further the ether was removed by evaporation and the residue was distilled under reduced

pressure. Yield: 170 g (65%) 11-methyl-spiro[5.5]undeca-1.8-diene-3-one; boiling point: 115—117°C/0.7 kPa.

The reaction product was dissolved in 150 ml ethanol after which 2 g 5% Pd/C catalyst was added. The mixture was hydrogenated at 400 kPa and 50°C until the theoretical amount of hydrogen was taken up. After removing the catalyst by filtration, the ethanol was removed by evaporation and the residue was distilled under reduced pressure. Yield: 162 g (93%) 7-methyl-spiro[5.5]undecane-3-one; boiling point: 117—118°C/0.7 kPa; $n_D^{25}$ = 1.4935.

## Example II

Preparation of 7.9-dimethyl-spiro[5.5]undecane-3-one.

A mixture of 69 g (0.5 mole) 2.4-dimethyl-3-cyclohexene carbaldehyde and 0.5 g p-toluene sulphonic acid was heated at 50—60°C. Over half an hour 35 g (0.5 mole) methyl-vinyl ketone was added after which the mixture was stirred for 4 hours at 60°C. Then 150 ml toluene was added and the formed water was distilled off azeotropically. After cooling down the mixture was poured out in 200 ml 10% soda solution. The layers were separated after which the organic layer was washed with brine and dried over $MgSO_4$. The toluene was removed by evaporation and the residue was distilled under reduced pressure. Yield: 40 g (42%) 7.9-dimethyl-spiro[5.5]undeca-1.8-diene-3-one; boiling point 117—120°C/O.7 kPa.

The reaction product was hydrogenated as mentioned in example I. Yield: 38.8 g (95%) 7.9-dimethyl-spiro[5.5]undecane-3-one; boiling point: 120—125°C/0.7 kPa $n_D^{25}$ = 1.4890.

This compound was also prepared according to the method mentioned in example I; the yield was 58%.

## Example III

A perfume composition for use in detergents was prepared according to the following recipe:

| | |
|---|---|
| α-Pentyl-cinnamaldehyde | 100 parts by weight |
| Terpineol | 90 parts by weight |
| 2-Phenylethanol | 75 parts by weight |
| Tetrahydromyrcenol | 70 parts by weight |
| Citronellol | 70 parts by weight |
| 4-Tert.butylcyclohexyl acetate | 70 parts by weight |
| Dihydromyrcenol | 60 parts by weight |
| Lavender oil | 55 parts by weight |
| 6-Acetyl-1-isopropyl-2.3.3.5-tetramethylindane | 50 parts by weight |
| 4-Acetoxy-3-pentyl-tetrahydropyran | 50 parts by weight |
| Tetrahydrolinalool | 40 parts by weight |
| 2-Methyl-3-(p-tert.butylphenyl)propanal | 40 parts by weight |
| γ-Methylionone | 40 parts by weight |
| Tricyclodecenyl propionate | 30 parts by weight |
| Heliotropine | 10 parts by weight |
| Coumarin | 10 parts by weight |
| Eugenol | 10 parts by weight |
| 7.9-Dimethyl-spiro[5.5]undecane-3-one (1e) | 30 parts by weight |
| | 900 parts by weight |

Example IV

A perfume composition for talcum powder was prepared according to the following recipe:

| | |
|---|---|
| 2-Phenylethanol | 100 parts by weight |
| γ-Methylionone | 100 parts by weight |
| Acetylcedrene | 70 parts by weight |
| Rosana NB 131* | 70 parts by weight |
| Cedar wood oil Virginia | 50 parts by weight |
| Benzyl acetate | 50 parts by weight |
| Lilas NB 146* | 50 parts by weight |
| Hydroxycitronellal | 40 parts by weight |
| Ylang-Ylang oil | 40 parts by weight |
| Geranium oil Bourbon | 25 parts by weight |
| 4-(4-Hydroxy-4-methylpentyl)-cyclohexene-3-carbaldehyde | 20 parts by weight |
| Musk Ambrette | 20 parts by weight |
| 3-Isocamphylcyclohexanol | 20 parts by weight |
| Dimethyl-benzyl-carbinyl acetate | 15 parts by weight |
| Coumarin | 5 parts by weight |
| 7-Methyl-spiro[5.5]undecane-3-one (1d) | 25 parts by weight |
| | 700 parts by weight |

*Fragrance bases available from Naarden International N.V.

**Claims**

1. A process for the preparation of perfume compositions, characterised in mixing one or more spiro(5.5)undecanones and/or undecenone having the following formula

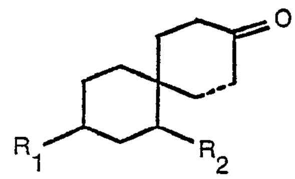

(1)

wherein $R_1$ and $R_2$ represent hydrogen or methyl and the dotted line represents a single or double bond, provided that $R_1$ and $R_2$ are both hydrogen when the dotted line is a double bond, with one or more conventional perfume components.

2. The process according to claim 1, characterised in using at least 0.01% by weight of the spiroundecanones and/or undecenone in the perfume composition.

3. A process for the preparation of perfumed products, characterised in that the products are mixed or brought in intimate contact with one or more spiro(5.5)undecanones and/or undecenone of formula 1.

4. A process for the preparation of perfumed products, characterised in that the products are mixed or brought in intimate contact with a perfume composition obtained according to claims 1 or 2.

5. 7-Methyl-spiro(5.5)undecane-3-one.

6. 7.9-Dimethyl-spiro(5.5)undecane-3-one.

**Patentansprüche:**

1. Verfahren zur Herstellung von Parfumkompositionen, gekennzeichnet durch Mischen eines oder mehrerer Spiro(5.5)undecanone und/oder -undecenone der folgenden Formel

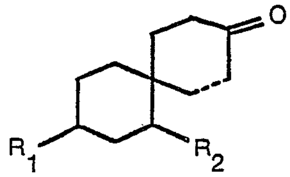

(1)

worin $R_1$ und $R_2$ ein Wasserstoffatom oder eine Methylgruppe sowie die gestrichelte Linie eine Einfach- oder eine Doppelbindung bedeuten, mit der Maßgabe, daß $R_1$ und $R_2$ jeweils ein Wasserstoffatom darstellen, wenn die gestrichelte Linie eine Doppelbindung bedeutet, mit einer oder mehreren üblichen Parfumkomponenten.

2. Verfahren nach Anspruch 1, gekennzeichnet durch den Einsatz von mindestens 0,01 Gewichtsprozent der Spiroundecanone und/oder -undecenone in der Parfumkomposition.

3. Verfahren zur Herstellung parfümierter Produkte, dadurch gekennzeichnet, daß die Produkte mit einem oder mehreren Spiro(5.5)undecanonen und/oder -undecenonen der Formel 1 gemischt oder in engen Kontakt gebracht werden.

4. Verfahren zur Herstellung parfümierter Produkte, dadurch gekennzeichnet, daß die Produkte mit einer gemäß Anspruch 1 oder 2 erhaltenen Parfumkomposition gemischt oder in engen Kontakt gebracht werden.

5. 7-Methylspiro(5.5)undecan-3-on.

6. 7,9-Dimethylspiro(5.5)undecan-3-on.

**Revendications**

1. Procédé de préparation de compositions de parfums, caractérisé en ce qu'on mélange un ou plusieurs spiro[5,5]undécanones et/ou undécénones répondant à la formule suivante:

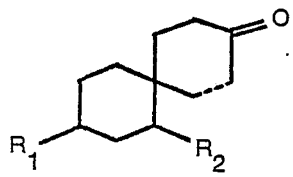

(1)

dans laquelle $R_1$ et $R_2$ représentent chacun un atome d'hydrogène ou un radical méthyle et la ligne en pointillé représente une liaison simple ou double, à la condition que $R_1$ et $R_2$ soient tous deux des atomes d'hydrogène quand la ligne en pointillé est une double liaison, avec un ou plusieurs composants classiques des parfums.

2. Procédé selon la revendication 1, caractérisé en ce qu'on utilise au moins 0,01% en poids de spiro-undécanones et/ou undécénones dans la composition de parfums.

3. Procédé de préparation de produits parfumés, caractérisé en ce qu'on mélange les produits ou on les met en contact intime avec un ou plusieurs spiro[5,5]undécanones et/ou undécénones de formule I.

4. Procédé de préparation de produits parfumés, caractérisé en ce qu'on mélange les produits ou on les met en contact intime avec une composition de parfum obtenue selon la revendication 1 ou 2.

5. 7-méthyl-spiro[5,5]undécan-3-one.

6. 7,9-diméthyl-spiro[5,5]undécan-3-one.